# EUROPEAN PATENT APPLICATION

(11) **EP 1 447 088 A1**
(43) Date of publication of application: **18.08.2004**
(21) Application number: 04003013.2
(22) Date of filing: 11.02.2004
(51) Int. Cl.: A61K 38/01

(54) **Vasodilator pharmaceutical preparation and health food composition**

(30) Priority: 13.02.2003 JP 2003035063
(71) Applicant: SHIRAKO CO., LTD., Tokyo, 134-8502 (JP)
(72) Inventor: Hagino, Hiroshi, Tokyo 152-0022 (JP)
(74) Representative: HOFFMANN - EITLE

(57) **Abstract**

This invention provides a novel material having a vasodilator action thereby suppressing or ameliorating various human diseases and disorders. A composition comprising, as an active ingredient, peptides obtained by hydrolyzing proteins such as proteins derived from a seaweed selected from laver, wakame, edible brown algae, sea tangle, chlorella and spirulina, proteins derived from a plant selected from soybean and sesame, proteins derived from a fish selected from bonito, mackerel, saury and horse mackerel, proteins derived from milk proteins selected from powdered skim milk and whey, proteins derived from an animal selected from cattle and swine, and collagen-like proteins derived from bovine collagen, porcine skin collagen and fish scale-derived collagen is used as a pharmaceutical composition and a health food composition thereby exhibiting a vasodilator effect by which various phenomena caused by a reduction in blood stream, such as stiff neck, headache and poor circulation, can be suppressed or ameliorated.

## Description

### Technical Field

The present invention relates to a pharmaceutical preparation and a health food composition having a vasodilator action thereby enabling suppression and amelioration of stiff neck, headache, poor circulation and functional depressions related thereto and in particular to the pharmaceutical preparation and health food composition comprising, as an active ingredient, peptides obtained by hydrolyzing various proteins.

### Background Art

Improvement of blood circulation leads to amelioration of symptoms such as stiff neck, poor circulation, headache and numbness of extremities, recovery from fatigue, and promotion of metabolism for peripheral tissues and hair. By promoting blood circulation, oxygen and nutriment spread sufficiently to peripheral tissues as a whole, while wastes such as carbon dioxide gas and lactic acid are collected, and thus the body is felt to be comfortable and warm just like a state after bathing, thus ameliorating symptoms such as fatigue, neuralgia and menopausal disorders.

There are some mechanisms promoting blood stream. One is to increase blood flow by improving the deformability of erythrocytes, and the other is to increase the amount of flowing blood by dilation of blood vessels. The prior art related to the former includes the prevention of deterioration in the deformability of erythrocytes by jujubes and an extract thereof (see JP-A 05-210639 and JP-A 07-61933) and also a report on collagen peptides reducing the viscosity of blood components such as erythrocytes, leucocytes and platelets thereby improving blood flow (see Japanese Patent No. 3197547).

There are also reports on the ameliorating effect of the following naturally occurring materials and extracts thereof on blood stream, but the mechanism ameliorating blood stream is not necessarily evident. Such known examples include saffron or an extract of saffron (see JP-A 10-287576), extracts of dandelions and mugwort (see JP-A 60-160856), a bouillon of bovine internal organs and bones (see JP-A 54-41354), burned materials of lotus root nodes and root hairs (see JP-A 53-133646), a water-soluble extract of red sweet potato (see JP-A 2001-145471), a theanine-containing material (see JP-A 2000-247878), rehmannia roots (see JP-A 2000-169385), and woundwort saponin (see JP-A 07-233191).

As an example in which vasodilatation was recognized, there is also a report on dilation of blood vessels in rabbit ears by peptides derived from sardine muscles (see Japanese Patent No. 2732056). However, these reports are those on only phenomena, and do not describe effectiveness and uses in humans.

In recent years, there has been demand for further development of blood stream improvers having various mechanisms improving blood stream.

### Summary of Invention

The object of the present invention is to provide a novel material having a vasodilator action thereby improving blood circulation to suppress or reduce stiff neck, poor circulation, headache, fatigue and menopausal disorders and to promote metabolism, and to further provide a health food and a pharmaceutical preparation having such action.

### Brief Description of Drawing

Fig. 1 is a graph showing experimental results of the vasodilator action of seaweed-derived peptides on rabbit ears.
Fig. 2 is a graph showing experimental results of the vasodilator action of seaweed-derived peptides on rabbit ears.
Fig. 3 is a graph showing experimental results of the vasodilator action of plant-derived peptides on rabbit ears.
Fig. 4 is a graph showing experimental results of the vasodilator action of fish-derived peptides on rabbit ears.
Fig. 5 is a graph showing experimental results of the vasodilator action of mammal-derived peptides on rabbit ears.
Fig. 6 is a graph showing experimental results of the vasodilator action of animal-derived peptides on rabbit ears.
Fig. 7 is a graph showing experimental results of the vasodilator action of collagen-derived peptides on rabbit ears.

### Disclosure of Invention

The present inventors have used ear vessels in rabbits to screen components based on peptides obtained by hydrolyzing various proteins, and as a result, they have found that these peptides have an effect of dilating blood vessels. That is, components containing peptides obtained by hydrolysis of various proteins by an acid or an alkali and/or an enzyme protease were orally administered as such or after purification into rabbits, to confirm dilatation of blood vessels.

The vasodilatation recognized in these rabbit ears has led to an increase in blood stream, and as a result, various effects such as an effect of depressing blood pressure, an ameliorating effect on stiff neck, poor circulation and headache, an effect of recovery from fatigue, an effect of suppressing and reducing menopausal disorders, an effect of restoring hair by promotion of metabolism, and an effect of improving skin conditions have been confirmed.

The protein hydrolysates recognized to have such effects include hydrolysates of proteins from plants such as soybeans and sesame seeds, hydrolysates of proteins from animals such as cattle and swine, hydrolysates of proteins from fishes such as bonito, mackerel, saury and horse mackerel, hydrolysates of proteins from seaweeds such as laver, wakame, edible brown algae, sea tangle etc. and micro-algae such as chlorella and spirulina, hydrolysates of milk proteins from powdered skim milk and whey proteins, hydrolysates of proteins derived from livestock products such as beef and pork, and hydrolysates of collagen proteins derived from bovine collagen, porcine collagen from porcine skin, and from fishes such as fish scales, and peptides contained in these hydrolysates were recognized to have a vasodilator effect.

Accordingly, the present invention relates to a vasodilator pharmaceutical preparation and health food composition comprising, as an active ingredient, peptides obtained by hydrolyzing the various proteins described above.

In hydrolysis of proteins in the present invention, acid or alkali decomposition and/or enzymatic decomposition with a protease is generally used. The acid or alkali may be an organic or inorganic acid or alkali, and it is preferable that the pH in acid hydrolysis is in the range of 1 to 4, and the pH in alkali hydrolysis is in the range of pH 8 to 13. The decomposition temperature and time are suitably established.

As the protease, use can be made of any generally used enzymes having a protease activity, such as pepsin, pancreatin, papain, Prolaser (Amano Pharmaceutical Co., Ltd.), Samoase (Yamato Kasei Co., Ltd.), Sumizyme AP, Sumizyme MP, Sumizyme FP (Shin-Nippon Kagaku Kogyo Co., Ltd.), etc. Reaction conditions such as the concentration of the enzyme used, reaction pH, reaction temperature, etc. may be selected such that the conditions are optimum for the enzyme used.

In acid and alkali hydrolysis, it is not necessarily easy to regulate the reaction so as to proceed uniformly. Accordingly, strict control of the reaction is necessary. Hydrolysis treatment with an acid or alkali can be used in combination with enzymatic hydrolysis. By acid or alkali treatment and subsequent treatment with a proteolytic enzyme, the molecular weight of the peptides is decreased thus increasing the ratio of low-molecular peptides. The low-molecular peptides are readily absorbed in digestive tracts and exhibit a high vasodilator effect, and it is thus desirable to increase the ratio of the low-molecular peptides in this manner. In the human digestive tracts, however, high-molecular peptides are degraded into low-molecular peptides by pepsin, trypsin and peptidases, and thus peptides other than the low-molecular peptides can be considered to exhibit the effect. It is however estimated that the low-molecular peptides exhibit the effect rapidly and reliably. Accordingly, the molecular weight of the peptides is preferably lower, but when the peptides are used as food, the same vasodilator effect can be recognized even if high-molecular peptides are contained therein.

Although the reaction products (peptide components) obtained by hydrolyzing each protein may be used as such, the peptide components may be concentrated and purified. The concentration and purification treatment can be carried out using desalting treatment by an electrodialysis membrane, desalting/concentration treatment with ion-exchange resin, discoloration, deodorization and concentration treatment with activated carbon and precipitation treatment with an organic solvent. The respective components may be used in a solution form, but can also be powdered by spray drying or lyophilization. The peptide components used may be further purified by chromatography.

It is desirable that various protein materials to be subjected to hydrolysis treatment are purified proteins. Various materials such as seaweeds, plant materials, fish, milk proteins, animal meat, and animal collagens can be used as such, but when the content of fat in the starting materials is high, fat remains in the resulting hydrolysate and undergoes oxidation, thus often generating nasty smells and tastes. Accordingly, starting materials from which oil was removed are desirably utilized. For example, soybeans or sesame used is preferably a bean cake or sesame seed cake with a lower content of oil after oil expression. Alternatively, oil can be removed after hydrolysis. It is also important to subject fish and livestock meat to treatment for removing oil. When seaweeds are used, on the other hand, the seaweeds can be subjected directly to hydrolysis without degreasing, and even after hydrolysis, a degreasing procedure is not particularly necessary because of a lower content of lipid components.

The vasodilator effect was confirmed with rabbit ears. That is, a sample was orally administered to rabbits, and a change in blood vessels in ears was confirmed with the naked eye, and from a photograph of the ears, the degree of vasodilatation was numerically expressed by using area calculation software. By this method, the vasodilator effects in the Examples shown later were confirmed.

In the present invention, it is estimated that as a result of the vasodilatation of blood vessels by protein hydrolysates, blood stream is increased thereby improving the transfer of nutriment to peripheral tissues and simultaneously improving the transfer of wastes. As a result, the protein hydrolysates can be used in humans for the purpose of suppressing and reducing stiff neck, headache and poor circulation. Further, the protein hydrolysates can improve blood circulation thereby improving physiological functions in which not only the circulatory organ system but also the nerve system, internal secretion system and immune system are involved, and thus the protein hydrolysates can be used for the purpose of suppressing and improving difficulty of sleep and menopausal disorders. Further, wastes such as carbon dioxide gas, lactic acid etc. can be suitably collected from peripheral tissues, while oxygen and nutriment can spread sufficiently to peripheral tissues, thus achieving recovery from fatigue and improvements in skin conditions, cosmetic effect and hair restoration effect. These effects are shown in test results in humans in the Examples.

The protein hydrolysates in the present invention can be used as a food additive added to general foods or as a health food composition or a pharmaceutical composition. The protein hydrolysates may be used in any forms such as an aqueous solution, a suspension, powder, and molded products, and their form is not particularly limited. Accordingly, the protein hydrolysates can be used in a wide variety of general foods, and can be provided as a health food and a pharmaceutical preparation in the form of capsules, tablets, powder, granules and drink. In this case, the protein hydrolysates can be used not only as a single tasting component but also in combination with functional materials such as other tasting components, excipients, stabilizers, Chinese medicines and herbs or their functional components, and the hydrolysates can be mixed with, and used in combination with, nutritive components such as vitamins and minerals and materials allowable as foods.

To exhibit these effects, the amount of the protein hydrolysates administered into humans is preferably 0.5 to 2000 mg/kg/day, more preferably 10 to 400 mg/kg/day. However, the dose is not limited to the above range because the type and degree of symptom are varied from individual to individual.

### Best Mode for Carrying Out the Invention

Hereinafter, embodiments of this invention are described in more detail by reference to the Production Examples and the Examples.

### Production Example 1

Laver peptides were prepared from laver proteins of seaweeds of the genus Porphyra in the following manner.

50 kg dry laver was suspended in 950 L hot water heated at 95°C and then boiled for 1 hour, and the broth was removed. Thereafter, 950 L water at 50°C was added thereto and adjusted to pH 2.0 with sulfuric acid, and 2 kg pepsin (Amano Pharmaceutical Co., Ltd.) was added thereto and reacted at 50°C for 24 hours under stirring. The resulting decomposed solution was adjusted to pH 5.0 with 1 N NaOH and kept at 50°C for 10 minutes to inactivate the pepsin. Then, extraction residues were removed by centrifugation (14000 r.p.m. for 20 minutes), and the supernatant was concentrated under reduced pressure and spray-dried to give a pepsin-digested laver product (sample 1).

1 kg of the pepsin-digested laver product was dissolved in distilled water, applied onto a Dowex-50 (H⁺) column, φ50 cmx200 cm (Bio-Rad) previously equilibrated with hydrochloric acid, then the column was washed with 120 L distilled water, and the adsorbed peptides were eluted with 2 N ammonia water. After the ammonia was removed with an evaporator, the sample was lyophilized to give 385 g of 90% laver peptides (sample 2).

### Production Example 2

10 L water was added to 2 kg laver powder (10- to 50-mesh size) which was then stirred and milled with an automatic mortar at room temperature for 1 hour, and the milled material was separated into laver extraction residues and a supernatant by centrifugation.

Ethanol was added to the resulting supernatant which was then left at -20°C for 12 hours to precipitate protein components and centrifuged to give 400 g water-soluble laver proteins as precipitates.

The laver proteins were subjected to acid hydrolysis and subsequent enzymatic decomposition in the following manner, to give low-molecular peptides. That is, 200 g laver proteins were dissolved in 1 L of 1 N hydrochloric acid and decomposed with the acid by heating at 100°C for 2 hours. Then, the solution was adjusted to pH 6.0 with sodium hydroxide, and after 5 g Sumizyme FP (Shin-Nippon Kagaku Kogyo Co., Ltd.) was added thereto, the solution was decomposed at 40°C for 8 hours. The decomposed solution was heated at 100°C for 20 minutes to inactivate the enzyme, and then concentrated under reduced pressure and treated in the same manner as for sample 2 in Production Example 1, to give 160 g laver peptides (sample 3).

### Production Example 3

Wakame peptides were prepared from wakame proteins in the following manner. 1 kg dry wakame seaweed was finely divided into powder of 35-mesh size, suspended in 20 L distilled water and milled with a wet mill. Then, the sample was centrifuged to give 5 L wakame protein-containing solution. The solution was concentrated into 1 L under reduced pressure, and then 8 L ethanol was added to the concentrate which was then left at -20°C for 12 hours to precipitate the proteins. The sample was then centrifuged, and the resulting precipitates were air-dried to give 100 g water-soluble wakame proteins.

50 g of the wakame proteins were dissolved in 500 ml phosphate buffer (pH 7.7), and 2.5 g thermolysin was added thereto and reacted at 40°C for 12 hours, to hydrolyze the proteins. After the reaction, the reaction solution was kept at 100°C for 20 minutes to inactivate the enzyme, and the reaction solution was lyophilized to give 35 g wakame peptides (sample 4).

Sea tangle peptides (sample 5) and edible brown alga peptides (sample 6) were obtained in the same treatment as described above.

### Production Example 4

Chlorella peptides and spirulina peptides were prepared respectively in the following manner. 50 g chlorella or spirulina powder was dissolved in 1 L of 0.5 N sodium hydroxide and decomposed with the alkali by heating at 80°C for 5 hours. Then, the sample was neutralized with hydrochloric acid and applied onto a Dowex-50 (H⁺) column (φ10 cmx65 cm) previously equilibrated with hydrochloric acid, then the column was washed with 5 L distilled water, and the adsorbed peptides were eluted with 2 N ammonia water. After the ammonia was removed with an evaporator, the sample was lyophilized to give 21 g chlorella peptides (sample 7) or 18 g spirulina peptides (sample 8).

### Production Example 5

As examples of the protein hydrolysates derived from plants, soybean peptides and sesame peptides were obtained from degreased bean cakes and degreased sesame seed cakes, respectively. That is, 1 kg degreased bean cakes or degreased sesame seed cakes were ground, then suspended in 5 L water and adjusted to pH 2.0 with sulfuric acid, and 40 g porcine stomach-derived pepsin (Amano Pharmaceutical Co., Ltd.) was added thereto and reacted at 50°C for 24 hours under stirring. After the reaction, the solids were separated by filtration, and the resulting supernatant was adsorbed onto Dowex-50 (H⁺), then washed with water, eluted with ammonia water, made free from ammonia, concentrated and lyophilized in the same manner as in Production Example 1, to give 200 g soybean peptides (sample 9) and 98 g sesame peptides (sample 10).

### Production Example 6

As examples of the protein hydrolysates derived from fish meats, peptides were obtained from fish meats of bonito, mackerel, saury and horse mackerel, respectively. 500 g fresh fish meat of bonito, mackerel, saury and horse mackerel were collected and milled, and after 1 L water was added thereto, the meat was thermally denatured by keeping it at 100°C for 10 minutes. The pH was adjusted to pH 2.0 with sulfuric acid, and 20 g pepsin (Amano Pharmaceutical Co., Ltd.) was added thereto and kept at 50°C for 16 hours. After the reaction was finished, the sample was adjusted to pH 5.0 with an aqueous sodium hydroxide solution and heated at 50°C for 10 minutes to inactivate the enzyme. The reaction solution was centrifuged, and the resulting supernatant was adsorbed onto Dowex-50 (H⁺), then washed sufficiently with water and eluted with 2 N ammonia water to give a peptide fraction. The peptide solution was concentrated under reduced pressure, made free from ammonia and lyophilized. By the above procedure, 30 g bonito-derived peptides (sample 11), 32 g mackerel-derived peptides (sample 12), 45 g saury-derived peptides (sample 13) or 33 g horse mackerel-derived peptides (sample 14) was obtained.

### Production Example 7

As examples of the protein hydrolysates derived from livestock milk, peptides were obtained in the following manner from powdered skim milk and whey respectively. 1 kg commercial powdered skim milk or commercial separated whey protein was suspended in 2 L warm water and adjusted to pH 7.5, and then 40 g Samoase (Yamato Kasei Co., Ltd.) was added thereto and reacted at 50°C for 16 hours. After the reaction, the reaction solution was heated at 100°C for 10 minutes to inactivate the enzyme, then adsorbed onto Dowex-50 (H⁺), washed with water and eluted with ammonia water to give a peptide fraction. The peptide solution was concentrated under reduced pressure, made free from ammonia and lyophilized to give 270 g powdered skim milk peptides (sample 15) or 333 g separated whey protein peptides (sample 16).

### Production Example 8

As examples of the protein hydrolysates derived from livestock meat, peptides were obtained in the following manner from beef and pork respectively.

1 kg minced beef with less oil was added to 2 L of 1 N hydrochloric acid and heated at 100°C for 2 hours. After cooling, the sample was adjusted to pH 2.0 with sodium hydroxide, and 40 g porcine stomach-derived pepsin (Amano Pharmaceutical Co., Ltd.) was added thereto and reacted at 50°C for 16 hours under stirring. After the reaction was finished, the reaction solution was neutralized to pH 5.0 with sodium hydroxide and kept at 50°C for 10 minutes to inactivate the enzyme. The reaction solution was centrifuged (3000 r.p.m., 10 minutes) to separate a supernatant. The oil phase in the supernatant was removed to the maximum degree, and the supernatant was applied onto Dowex-50 (H⁺), then sufficiently washed with water and eluted with 2 N ammonia water to give a peptide fraction. The peptide solution was concentrated under reduced pressure, made free of ammonia and lyophilized to give 343 g beef-derived peptides (sample 17). 318 g pork-derived peptides (sample 18) were also obtained in the same manner as described above.

### Production Example 9

As examples of the collagen hydrolysates, peptides were obtained in the following manner from porcine skin-derived glue and fish scales respectively.

2 kg glue derived from a commercial porcine skin was ground and suspended in 4 L water previously made acidic (pH 3.0) with hydrochloric acid, and stirred at room temperature for 3 days. Thereafter, the suspension was adjusted to pH 2 with hydrochloric acid, and 40 g porcine stomach-derived pepsin (Amano Pharmaceutical Co., Ltd.) was added thereto and reacted at 50°C for 16 hours. After the reaction was finished, the reaction solution was neutralized to pH 5.0 with sodium hydroxide and kept at 50°C for 10 minutes to inactivate the enzyme. Thereafter, the reaction suspension was centrifuged to give a supernatant which was then applied onto a Dowex-50 (H⁺) column, then washed with water and eluted with 2 N ammonia water to give a peptide fraction. The peptide eluate was concentrated under reduced pressure and lyophilized to give 720 g glue-derived collagen peptides (sample 19).

Separately, 1 L commercial fish scale-derived 50% collagen solution (Timely Co., Ltd.) was decomposed with pepsin, purified with the cation-exchange resin and powdered by lyophilization in the same manner as described above, to give 265 g fish scale-derived collagen peptides (sample 20).

The vasodilator effects of the samples obtained in the Production Examples above were confirmed and then the samples were used as shown in the following examples.

### Example 1

Nine-week-old male rabbits (Slc : JW·CSK) each weighing 1.5 to 1.8 kg were preliminarily bred for 1 week and then subjected to experiment. An animal breeding chamber was maintained at a temperature of 22°C±1°C in 50% humidity in a bright (12 hours)/dark (12 hours) cycle, and the animals were allowed feed Lab Diet 5L95 (Nippon SLC) and drinking water ad libitum. Before administration of a sample, the animals were fasted for 2 hours. The sample, 1000 mg/kg body weight, was dissolved in 3 to 10 ml physiological saline and administered forcibly through an oral probe into the animals.

The state of blood vessels in the rabbit was examined in the following manner. The rabbit was fixed to a blood collection box, and when the rabbit became quiet to show no change in ear vessels, a photograph of the ear was taken with a digital camera and this point in time was regarded as 0 minute. From 10 minutes after the sample was administered, a photograph of the ear was taken repeatedly at intervals of 10 minutes until the vasodilatation became calm. The sectional area of a vessel in the photograph was calculated with area calculation software LIA32 and numerically expressed.

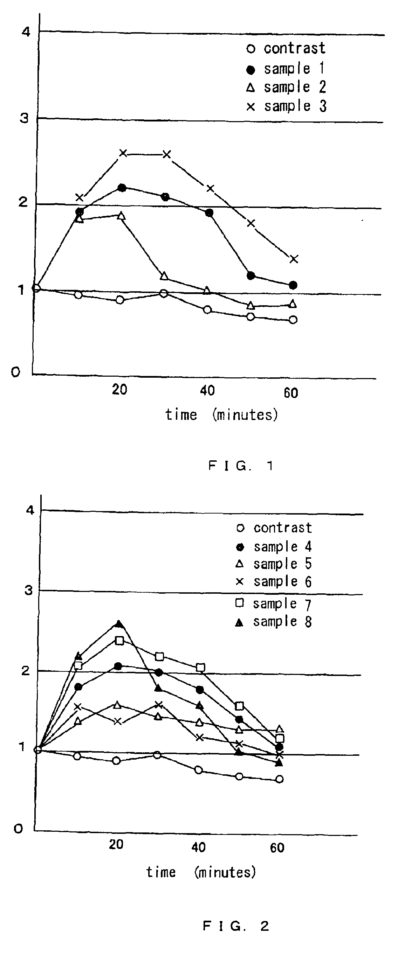

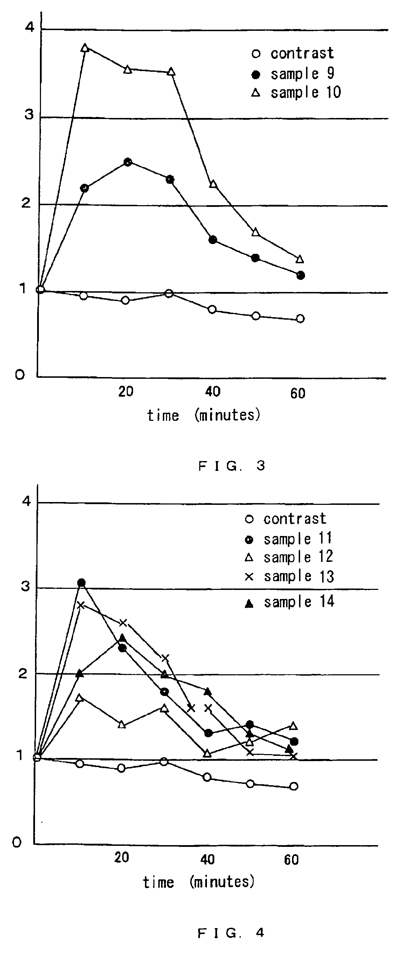

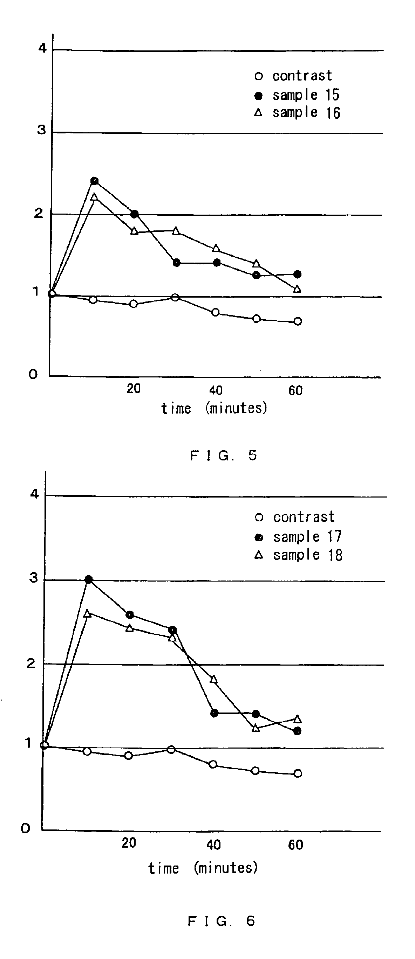

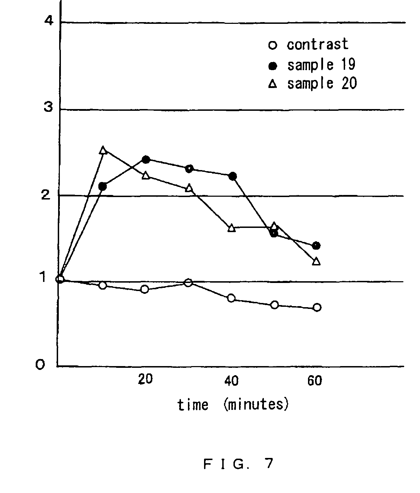

Samples 1 to 20 obtained in Production Examples 1 to 9 were measured as described above. The results are shown in graphs in Figs. 1 to 7. In the graph, the sectional area of a blood vessel measured is shown on the ordinate wherein the area at 0 minute is 1, and the change of this area with time is illustrated. As a result, all samples were recognized to exhibit a vasodilator action in the rabbit ears.

### Example 2

1.2 g granules consisting of 75 weight% sample 1, 15% reducing maltose syrup, 5.8% dextrin, 0.9% citric acid, 2.3% perfume and 1.0% sweetener (stevia) were enveloped as one package and used as a pharmaceutical preparation. Separately, tablets each weighing 300 mg, consisting of 88 weight% sample 1, 9% reducing maltose and 3% sucrose fatty ester, were produced.

### Example 3

Tablets each weighing 300 mg, consisting of 80 weight% of each of samples 2 to 20, 10 weight% reducing maltose syrup, 7 weight% dextrin and 3 weight% sucrose fatty ester, were produced.

### Example 4

Two packages of the granules in Example 2 were administered (one package in the morning and one package in the evening) every day for 30 days into each of 20 volunteers suffering from stiff neck. After 30 days, each volunteer was allowed to fill in a questionnaire. The result is shown in Table 1. As a result, 70% volunteers dissolved or reduced stiff neck, to indicate an effect on them.

**Table 1**

| Symptoms | Number of persons |
|---|---|
| Dissolved stiff neck | 5 |
| Reduced stiff neck | 9 |
| Not changed | 6 |
| Severer stiff neck | 0 |

### Example 5

Six tablets containing sample 2 in Example 3 were administered every day for 30 days into each of 10 volunteers suffering from stiff neck. After 30 days, each volunteer was allowed to fill in a questionnaire. The result is shown in Table 2. The result indicated that 60% volunteers dissolved or reduced stiff neck.

**Table 2**

| Symptoms | Number of persons |
|---|---|
| Dissolved stiff neck | 2 |
| Reduced stiff neck | 4 |
| Not changed | 4 |
| Severer stiff neck | 0 |

### Example 6

Six tablets containing sample 3 in Example 3 were administered every day for 30 days into each of 10 volunteers suffering from stiff neck. When each volunteer was allowed to fill in a questionnaire after 30 days, 3 person dissolved stiff neck, 3 persons reduced stiff neck, and the other 4 persons were not changed.

### Example 7

Six tablets in Example 2 were administered every day for 2 weeks into each of 20- to 40-year-old 20 female volunteers suffering from poor circulation, and after 2 weeks, each volunteer was allowed to fill in a questionnaire. The result is shown in Table 3. The result indicated that 13 volunteers dissolved or reduced poor circulation.

**Table 3**

| Symptoms | Number of persons |
|---|---|
| Dissolved poor circulation | 3 |
| Reduced poor circulation | 10 |
| Not changed | 7 |
| Severer poor circulation | 0 |

### Example 8

Six tablets containing sample 10 in Example 3 were administered every day for 2 weeks into each of 20- to 40-year-old 10 female volunteers suffering from poor circulation, and after 2 weeks, each volunteer was allowed to fill in a questionnaire. The result is shown in Table 4. The result indicated that 6 volunteers dissolved or reduced poor circulation.

**Table 4**

| Symptoms | Number of persons |
|---|---|
| Dissolved poor circulation | 2 |
| Reduced poor circulation | 4 |
| Not changed | 4 |
| Severer poor circulation | 0 |

### Example 9

The temperature of a fingertip after administration of tablets containing each of samples 1 to 20 described in Examples 2 and 3 was measured. In this test, 10 volunteers were selected from those experiencing an effect of dissolving or reducing poor circulation in Example 7, and examined for several days. Those without breakfast entered a room regulated at 20°C in 50% humidity, and after 1 hour, given 6 tablets containing each of samples 1 to 20, together with 50 ml water, and 30 minutes after this administration, a change in the temperature of their fingertip was measured with a thermistor thermometer, to determine an increase in the temperature from 0 hour. Two volunteers were examined for each sample, and the average was recorded.

**Table 5**

| Sample | Increased temp. (°C) | Sample | Increased temp. (°C) |
|---|---|---|---|
| 1 | 0.3 | 11 | 0.5 |
| 2 | 0.5 | 12 | 0.2 |
| 3 | 0.4 | 13 | 0.1 |
| 4 | 0.3 | 14 | 0.3 |
| 5 | 0.2 | 15 | 0.3 |
| 6 | 0.1 | 16 | 0.4 |
| 7 | 0.5 | 17 | 0.5 |
| 8 | 0.4 | 18 | 0.4 |
| 9 | 0.4 | 19 | 0.2 |
| 10 | 0.5 | 20 | 0.2 |

### Example 10

Two packages containing granules in Example 2 were administered every day for 30 days into each of 15 volunteers suffering from migraine headache. After 30 days, each volunteer was allowed to fill in a questionnaire. As shown in Table 6, the result indicated that 8 volunteers dissolved or reduced headache.

**Table 6**

| Symptoms | Number of persons |
|---|---|
| Dissolved headache | 2 |
| Reduced headache | 6 |
| Not changed | 7 |
| Severer headache | 0 |

### Example 11

Two packages containing granules in Example 2 were administered every day for 30 days into each of 55- to 63-year-old 5 women having menopausal high blood pressure. Together with this preparation, estrogen was given to any persons. As a result, the blood pressure in any persons was reduced to the normal range, and the body was left to be warmer than when they had used estrogen only. It was also recognized that menopausal disorders was reduced.

### Example 12

Five athletes were allowed to run for 1500 m with all their strength, and before the running and 5 minutes after the running, blood was collected, and the lactic acid level in blood was measured. On the next day, they were given two packages of granules in Example 2, and then allowed to run for 1500 m with all their strength, and the lactic acid level in blood was measured in the same manner as on the previous day. The result is shown in Table 7. The lactic acid level in blood in the athletes given the granules was lower than that without administering the granules.

**Table 7**

| Blood lactic acid level (mmol/l) | | | | |
|---|---|---|---|---|
| | Not given the granules | | Given the granules | |
| | before running | after running | before running | after running |
| A | 1.0 | 10.5 | 1.1 | 9.0 |
| B | 1.1 | 10.8 | 1.0 | 8.3 |
| C | 0.9 | 11.1 | 1.0 | 7.8 |
| D | 1.1 | 12.1 | 0.9 | 7.6 |
| E | 1.2 | 12.3 | 1.2 | 8.1 |

### Example 13

Six tablets in Example 2 were given to each of 30- to 35-year-old 20 women, and after 1 month, a change in skin conditions was evaluated by themselves (checks in plural items were allowable). As a result, the following skin-improving effects were recognized.

Their complexion was improved (14 persons), skin gloss was improved (12), skin moistness was improved (9), skin roughness was eliminated (9), cosmetics could stick more easily to the skin (8), and skin elasticity was improved (7).

### Example 14

Six tablets containing sample 4 in Example 3 were administered into each of 30- to 35-year-old 10 women, and after 1 month, a change in skin conditions was evaluated by themselves (checks in plural items were allowable). As a result, the following skin-improving effects were recognized.

Their complexion was improved (7 persons), skin gloss was improved (6), skin roughness was eliminated (4), skin moistness was improved (4) and cosmetics could stick more easily to the skin (3).

### Example 15

Two packages of granules in Example 2 were given every day for 3 months to each of twenty men aware of loss of a lot of hair, and thereafter, they were questioned about a change in hair loss. The result indicated that 8 persons recognized a reduction in hair loss, and 7 persons had thicker hair.

### Industrial Applicability

According to the present invention, a composition comprising peptides obtained by hydrolyzing various proteins such as seaweed-derived proteins, plant-derived proteins, fish-derived proteins, milk proteins, animal-derived proteins and collagen-like proteins is used in a pharmaceutical preparation and a health food thereby exhibiting a vasodilator effect by which stiff neck, headache, poor circulation and functional depressions related thereto can be suppressed or ameliorated.

## Claims

1. A vasodilator pharmaceutical composition comprising, as an active ingredient, peptides obtained by hydrolyzing proteins derived from a seaweed selected from laver, wakame, edible brown algae, sea tangle, chlorella and/or spirulina, peptides obtained by hydrolyzing proteins derived from a plant selected from soybean and/or sesame, peptides obtained by hydrolyzing proteins derived from a fish selected from bonito, mackerel, saury and/or horse mackerel, peptides obtained by hydrolyzing proteins derived from an animal selected from cattle and/or swine, or peptides obtained by hydrolyzing collagen-like proteins derived from bovine collagen, porcine skin collagen and/or fish scale-derived collagen.

2. A vasodilator health food composition comprising, as an active ingredient, peptides obtained by hydrolyzing proteins derived from a seaweed selected from laver, wakame, edible brown algae, sea tangle, chlorella and spirulina, peptides obtained by hydrolyzing proteins derived from a plant selected from soybean and/or sesame, peptides obtained by hydrolyzing proteins derived from a fish selected from bonito, mackerel, saury and/or horse mackerel, peptides obtained by hydrolyzing proteins derived from milk proteins selected from powdered skin milk and/or whey, peptides obtained by hydrolyzing proteins derived from an animal selected from cattle and/or swine, or peptides obtained by hydrolyzing collagen-like proteins derived from bovine collagen, porcine skin collagen and/or fish scale-derived collagen.

3. Use of peptides obtained by hydrolyzing proteins derived from a seaweed selected from laver, wakame, edible brown algae, sea tangle, chlorella and/or spirulina, peptides obtained by hydrolyzing proteins derived from a plant selected from soybean and/or sesame, peptides obtained by hydrolyzing proteins derived from a fish selected from bonito, mackerel, saury and/or horse mackerel, peptides obtained by hydrolyzing proteins derived from an animal selected from cattle and/or swine, or peptides obtained by hydrolyzing collagen-like proteins derived from bovine collagen, porcine skin collagen and/or fish scale-derived collagen for the preparation of a medicament useful in the treatment of a stiff neck, poor circulation, headache, fatigue, or menopausal disorders.

4. Use of peptides obtained by hydrolyzing proteins derived from a seaweed selected from laver, wakame, edible brown algae, sea tangle, chlorella and/or spirulina, peptides obtained by hydrolyzing proteins derived from a plant selected from soybean and/or sesame, peptides obtained by hydrolyzing proteins derived from a fish selected from bonito, mackerel, saury and/or horse mackerel, peptides obtained by hydrolyzing proteins derived from an animal selected from cattle and/or swine, or peptides obtained by hydrolyzing collagen-like proteins derived from bovine collagen, porcine skin collagen and/or fish scale-derived collagen as a food additive.

5. Use of peptides obtained by hydrolyzing proteins derived from a seaweed selected from laver, wakame, edible brown algae, sea tangle, chlorella and/or spirulina, peptides obtained by hydrolyzing proteins derived from a plant selected from soybean and/or sesame, peptides obtained by hydrolyzing proteins derived from a fish selected from bonito, mackerel, saury and/or horse mackerel, peptides obtained by hydrolyzing proteins derived from an animal selected from cattle and/or swine, or peptides obtained by hydrolyzing collagen-like proteins derived from bovine collagen, porcine skin collagen and/or fish scale-derived collagen in cosmetics for the improvement of skin conditions and for hair restoration.
